(19) Europäisches Patentamt
European Patent Office
Office européen des brevets

(11) Publication number: 0 200 923 B1

# (12) EUROPEAN PATENT SPECIFICATION

(45) Date of publication of patent specification: 20.03.91 (51) Int. Cl.5: C07C 209/60

(21) Application number: 86104554.0

(22) Date of filing: 03.04.86

## (54) Preparation of amines from olefins using ammonium halide catalysts.

(30) Priority: 18.04.85 US 724737
06.03.86 US 836874

(43) Date of publication of application:
12.11.86 Bulletin 86/46

(45) Publication of the grant of the patent:
20.03.91 Bulletin 91/12

(84) Designated Contracting States:
AT BE CH DE FR GB IT LI LU NL SE

(56) References cited:
EP-A- 0 158 716
US-A- 2 706 730

CHEMICAL ABSTRACTS, vol. 96, no. 21, 24th May 1982, page 662, column 2, abstract no. 180708a, Columbus, Ohio, US; F. ABE et al.: "Reaction of mixtures of higher alcohols and their aluminium alcholates with ammonia. Addition of aluminium oxide"

(73) Proprietor: ATOCHEM NORTH AMERICA, INC.
(a Pennsylvania corp.)
Three Parkway
Philadelphia Pennsylvania 19102(US)

(72) Inventor: Gardner, David Milton
1525 Green Hill Road
Collegeville, PA 19426(US)
Inventor: Mc Elligott, Paul Joseph
1158 Wheatsheaf Lane
Abington, PA 19001(US)
Inventor: Clark, Roger Thomas
531 Neiman Road
Pottstown, PA 19464(US)

(74) Representative: Kraus, Walter, Dr. et al
Patentanwälte Kraus, Weisert & Partner
Thomas-Wimmer-Ring 15
W-8000 München 22(DE)

EP 0 200 923 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for producing amines by the reaction, at elevated temperature, of ammonia or a primary or secondary amine with an olefin in the presence of an ammonium halide-containing catalyst. Preferably, the catalyst is used in conjunction with a specified transition metal promoter.

The reaction of ammonia with olefins in the vapor phase over a variety of catalysts including certain metal salts and oxides and mixtures of metal salts and oxides deposited on inert, porous supports is known in the art. Such processes are described for instance in US-A-2 479 879, 2 417 892, 2 417 893, 2 381 470, 2 381 709, 2 392 107, 2 398 899 and 3 412 158. The products of such reaction are reportedly mixtures of amines, nitriles, polyamines, polyolefins and degradation products. The low selectivity to amines is the primary reason this technology has never been commercialized.

In US-A-4 483 757 there is disclosed a liquid phase, photochemical process of reacting an olefin with ammonia or a primary or secondary amine in the presence of ammonium iodide or ammonium bromide catalyst. The reaction is carried out under the influence of light having spectral emissions in excess of 160 nm. and at a temperature ranging from the freezing temperature of the reactants up to about 40° C.

US-A-2 706 730 describes the synthesis of pyridine derivatives from alkenyl alkyl ethers and ammonia and particularly the formation of 2-methyl-5-ethylpyridine by reaction of methyl vinyl ether with ammonia.

Chemical Abstracts, Vol. 96, 1982, page 662, column 2, Abstract No. 180 708a describe the reaction of mixtures of higher alcohols and their aluminum alcoholates with ammonia.

This invention provides a process for producing amines which is characterized by reacting ammonia or a primary or secondary amine with an aliphatic monoolefin having from 2 to 8 carbon atoms at a temperature ranging from 170° to 450° C, at a pressure of from about atmospheric to 587.1 bar (8500 psig) and in the presence of an ammonium halide-containing catalyst. The process of the present invention is further improved by the incorporation of certain non-catalytic transition metal compounds (promoters) as a part of the catalyst system to thereby significantly increase catalyst activity.

This invention is directed to an improved process for the preparation of aliphatic and aromatic amines which comprises reacting an aliphatic monoolefin having from 2 to 8 carbon atoms with ammonia or a primary or secondary amine. The reaction is carried out at a temperature and pressure sufficient to effect rapid formation of amine, but mild enough to minimize formation of olefin polymer, polyamines, and other undesirable side products.

Examples of the monoolefins suitable for producing amines by this process are ethylene, propylene, 1-butene, isobutylene, 1-pentene, 2-pentene, 2-methyl-2-butene, 1-hexene, 2-hexene, 3-hexene, 3-methyl-1-pentene, 1-heptene, 2-heptene, 1-octene, 2-octene, and 3-ethyl-2-hexene. Monoolefins having from 2 to 4 carbon atoms are preferred.

Specific examples of the N-H containing reactants which are used in this process include ammonia, methylamine, dimethylamine, ethylamine, diethylamine, n-propylamine, di(n-propyl)amine, isopropylamine, di(isopropyl)amine, n-butylamine, di(n-butyl)amine isobutylamine, di(isobutyl)amine, the pentyl and higher alkylamines; cycloaliphatic amines eg., cyclohexylamine; aromatic amines, eg., aniline; the N-alkylanilines, diphenylamine, the naphthylamines, and the toluidines; heterocyclic amines, eg., pyrrolidine, morpholine, and piperidine; substituted amines, eg., alkanolamines; and polyamines, eg., ethylenediamine and 1,6-hexanediamine. The preferred N-H containing reactant, because of its commercial significance, is ammonia.

In the process of this invention, ammonia or an amine is reacted with an olefin at temperatures over a range of 170° to 450° C. The preferred temperature range is 250° to 350° C. Lower temperatures result in lower conversion and higher temperatures result in lower selectivity to the desired amine product.

Pressures in the range of atmospheric to 587.1 bar (8,500 psig) are useful for carrying out this invention. Pressures in the range of 83.8 bar (1,200 psig) to 276.9 bar (4,000 psig) are preferred.

The molar ratio of ammonia or amine to olefin can be from 20:1 to 0.2:1 with the preferred range of from 4:1 to 1:1.

An important factor in achieving the desired results of high amine selectivity and low byproduct formation in this process is the use of ammonium halide-containing catalysts. The catalyst may be ammonium fluoride, ammonium chloride, ammonium bromide, ammonium iodide or an alkyl- or aryl-substituted ammonium halide such as ethylammonium iodide. If the reaction is carried out with ammonia, the ammonium halide-containing compositions employed as catalysts herein include halide compounds which react with ammonia under the process conditions disclosed to form the corresponding ammonium halide which will in turn catalyze the reaction to form amines from olefins.

Examples of such halide compounds are alkyl halides and reactive metal halides, e.g., zinc chloride, cobalt (II) chloride, chromium (III) chloride, copper (II) chloride, copper (II) iodide, boron fluoride, ammonium fluoroborate, ammonium hexafluorophosphate, ammonium hexafluorogermanate, tin (II) chloride, ferric chloride, and silicon halides. The preferred catalysts of this invention are ammonium iodide, ammonium bromide, and ammonium chloride. The halide catalysts can be used individually or in combinations with each other to improve conversions and yields.

The foregoing halides can be advantageously used in this process dissolved or suspended in a suitable solvent or mounted or deposited on a suitable solid, porous, inert catalyst support. For example, such supports include alumina, silica-alumina, silica, carbon, calcium aluminate, kieselguhr, titania, zirconia, magnesia, silica-magnesia, various clays and refractory materials. Examples of catalyst solvents include water, ethanol, ethylene glycol, t-butylalcohol, and various amines, olefins and hydrocarbons.

For homogeneous liquid phase catalyst systems, where the halide is dissolved in a solvent, the halide can be used in any catalytically effective amount with respect to the amine or ammonia reactant; the preferred amount ranging from at least 0.01 mole/mole of amine or ammonia up to the limit of solubility of the halide in a reaction media. Reaction times depend on the catalyst concentration, reaction conditions and on the reactants, but generally fall in a range of from 1 minute to 5 hours.

For a continuous heterogenous vapor phase catalyst, where the halide is mounted or deposited on a solid support, the reaction time is defined in terms of the gas hourly space velocity (GHSV) which has units of, volume of gas per volume of catalyst in unit time, i.e., (cc gas at STP)/(cc catalyst) ($hours^{-1}$). STP means standard temperature (25 °C) and pressure 0.98 bar (1 atm). The gas hourly space velocity can be from 300 to 15,000 $hr^{-1}$. As the space velocity is increased toward the upper end of the range, particularly above 5,000 hr,$^{-1}$ conversion falls off. On the other hand, as the space velocity approaches the lower level of 300 $hr^{-1}$, the reaction mixture approaches equilibrium hence the production rate falls to zero. Where the olefin is isobutylene, typical conversions of from about 14% to 22% are achieved at space velocities of about 2,300 to 550 $hr^{-1}$.

The non-catalytic transition metal promoters which will significantly increase the catalytic activity of the ammonium halide catalyst include the transition metal halides and ammonium salts as well as the sodium salts of inorganic oxyacids. Examples of these compounds include chromium chloride ($CrCl_3$), chromium iodide ($CrI_3$), copper iodide ($CuI$), zinc chloride ($ZnCl_2$), ammonium phosphomolybdate [$(NH_4)_3PO_4 \cdot 12MoO_3 \cdot H_2O$], ammonium vanadate ($NH_4VO_3$), sodium stannate ($Na_2SnO_3$) and cobalt chloride hexahydrate ($CoCl_2 \cdot 6H_2O$).

The promoter is used in admixture with the catalyst whereby there is intimate contact between the two substances. If the promoter is deposited on a solid support, it should be on the same support which bears the catalyst.

The promoter is used in any amount which will improve the performance of the ammonium halide catalyst in this process, the preferred amount of promoter ranging from 1 to 0.01 parts, more preferable from 0.1 to 0.05 parts by weight of promoter per part by weight of active catalyst.

The preferred catalyst-promoter combination is ammonium iodide-chromium chloride.

In accordance with this process, the amination of the olefin with ammonia or an amine (primary or secondary) provides amines of the next level of substitution. Thus, ammonia yields principally primary amines, primary amines yield secondary amines and secondary amines yield tertiary amines. The general reaction for the process is illustrated by the following equation:

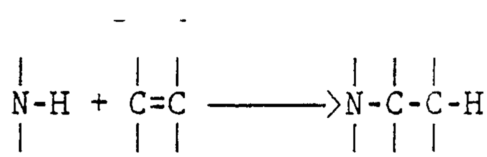

Two methods of carrying out this reaction have been investigated. In the first or static method, the catalyst, olefin, ammonia and solvent, if employed, are placed in an autoclave, and heated electrically. The reactant mixture is agitated (stirring or shaking) under autogenous pressure for a prescribed period of time. The autoclave is cooled and the products recovered. In the second or continuous method, the catalyst may be either neat or supported on an inert support, such as described above, and the reactants are passed over the catalyst at elevated temperatures and pressure using a suitably designed apparatus.

The following examples illustrate the preferred embodiments of this invention.

Percentages are mole percents, temperatures are in degrees centigrade, and pressures are in bar (psig)

(i.e, above atmospheric pressure). Conversions and yields given in these examples are based on the isolated products. Conversion is defined as the percentage of olefin charged to the reaction zone which has undergone reaction. Yield is defined as the percent of olefin reacted which has formed the indicated product(s). Major products were identified by gas chromatographic and mass-spectroscopic

analytical methods and when necessary, were confirmed by distillation followed by infrared spectrophotometry and nuclear magnetic resonance spectroscopy. Those minor products which are not identified are assumed to have the same gas chromatographic response factor as the major amine product.

It has been observed when carrying out the process described herein, that in the absence of a catalyst or in the presence of an ineffective catalyst, that no amine products are formed.

Example 1

A mixture of 19.3g (1.1 moles) anhydrous ammonia, 14.8g (0.53 mole) of ethylene, 14.5g (0.56 mole) ammonium iodide and 81g of water were charged to a 300cm$^3$ stainless steel autoclave. The autoclave was heated to 330°C and held there for five hours with agitation; the maximum system pressure was 194.1 bar (2,800 psig). A total of 7.0g of monoethylamine, 1.1g of diethylamine, 0.02g of triethylamine, and 1.1g of ethyl alcohol were obtained, corresponding to an ethylene conversion of 43.9% and a combined ethylamines yield of 83.8% and ethyl alcohol yield of 11.0%. The balance consisted of approximately 0.33g of a hydrocarbon oil.

Example 2

A mixture of 34.1g (2.0 moles) anhydrous ammonia, 28.0g (1.0 mcle) of ethylene and 14.5g (0.24 mole) of ammonium iodide were charged to a 300cm$^3$ stainless steel autoclave. The autoclave was heated to 368-370°C and held there for five hours with agitation; the maximum system pressure was 352.7 bar (5,100 psig). A total of 4.2g of monoethylamine, 0.1g of diethylamine, and detectable quantities of triethylamine were obtained, corresponding to an ethylene conversion of 10.2% and a combined ethylamines yield of 92.2%. The balance consisted of approximately 0.2g of a hydrocarbon oil.

Example 3

A mixture of 8.9g (0.52 mole) anhydrous ammonium, 7.5g (0.27 mole) of ethylene and 14.5g (0.10 moles) of ammonium iodide were charged to a 300cm$^3$ stainless steel autoclave. The autoclave was heated to 380-381°C and held there for five hours with agitation; the maximum system pressure was 1,125 psig. A total of 0.46g of monoethylamine and a trace of diethylamine were obtained, corresponding to an ethylene conversion of 4.0% and a combined ethylamines yield of 100%.

In Examples 4-12 following, the autoclave was heated to 350°C and held there for five hours with continuous agitation of the reactants unless specified otherwise.

Example 4

A mixture of 34.1g (2.0 moles) anhydrous ammonia, 28.0g (1.0 mole) of ethylene and 5.4g (0.10 mole) of ammonium chloride were charged to a 300cm$^3$ stainless steel autoclave. The maximum system pressure was 352.7 bar (5,100 psig). A total of 0.73g of monoethylamine and trace amounts of diethylamine were obtained, corresponding to an ethylene conversion of 1.6% and an ethylamine yield of 100%.

Example 5

A mixture of 34.1g (2.0 moles) anhydrous ammonia, 28.0g (1.0 mole) of ethylene and 13.2g (0.13 mole) of ammonium bromide were charged to a 300cm$^3$ stainless steel autoclave. The maximum system pressure was 414.8 bar (6,000 psig). A total of 0.2g of monoethylamine and 0.2g of a hydrocarbon oil were obtained, corresponding to an ethylene conversion of 1.2% and an ethylamine yield of 38%.

Example 6

A mixture of 34.1g (2.0 moles) anhydrous ammonia, 28.0g (1.0 mole) of ethylene and 3.7g (0.10 mole) of ammonium fluoride were charged to a 300cm$^3$ stainless steel autoclave. The maximum system pressure was 318.2 bar (4,600 psig). Trace amounts of monethylamine were obtained.

Example 7

A mixture of 18.5g (1.1 moles) of anyhydrous ammonia, 27.9g (0.5 mole) of isobutylene, 78.7g of water and 43.6g (0.10 mole) of ammonium iodide were charged to a 300cm$^3$ stainless steel autoclave. The autoclave was heated to 315°C and held there for two hours with agitation; the maximum system pressure wad 201.0 bar (2,900 psig). A total of 6.4g of t-butylamine, 8.1g of t-butyl alcohol and 0.3g of a hydrocarbon oil were obtained, corresponding to an isobutylene conversion of 27.2% and a t-butylamine yield of 65%, t-butyl alcohol yield of 31% and oil yield of 3.9%.

Example 8

A mixture of 18.1g (1.1 moles) of anhydrous ammonia, 27.5g (0.5 mole) of cis, trans-2-butenes, 79.1g of water and 43.5g (0.10 mole) of ammonium iodide were charged to a 300cm$^3$ stainless steel autoclave. The autoclave was heated to 335° C and held there for five hours with agitation; the maximum system pressure was 214.8 bar (3,100 psig). A total of 3.3g of mono-sec-butylamine, 0.01g of di-sec-butylamine, 0.42g of mono-n-butylamine, 1.29g of sec-butyl alcohol, 0.06g n-butyl alcohol, 0.35g methylethylketone and 0.93g of a hydrocarbon oil were obtained, corresponding to a 2-butene conversion of 19.9%, an amines yield of 52.1%, an alcohol yield of 25.9%, MEK yield of 5.0% and an oil yield of 17.0%.

Example 9

A mixture of 17.0g (1.0 mole) anhydrous ammonia, 28.0g (0.5 mole) of isobutylene and 16.0g (0.3 mole) ammonium chloride were charged to a 300cm$^3$ stainless steel autoclave. The autoclave was heated to 315° C and held there for 2 hours with agitation. The maximum system pressure was 201.0 bar (2,900 psig). A total of 0.82g of t-butylamine was obtained, corresponding to an isobutylene conversion of 2.2%, a t-butylamine yield of 43% and a hydrocarbon oil yield of 57%.

Example 10

A mixture of 17.0g (1.0 mole) of anhydrous ammonia, 21.0g (0.5 mole) of propylene and 16.0g (0.3 mole) of ammonium chloride were charged to a 300cm$^3$ stainless steel autoclave. The autoclave was heated to 335° C and held there for with agitation. The maximum system pressure was 194.1 bar (2,800 psig). A total of 2.6g of monoisopropylamine and 0.54g of diisopropylamine was obtained, corresponding to a propylene conversion of 10.9%, a combined isopropyl- and diisopropylamine yield of 62.5% and a hydrocarbon oil yield of 89.1%.

Example 11

A mixture of 16.9g (1.0 mole) of anhydrous ammonia, 21.1g (0.5 mole) of propylene, 50.0g water and 43.5g (0.3 mole) of ammonium iodide were charged to a 300cm$^3$ stainless steel autoclave. The autoclave was heated to 335° C and held there with agitation. A total of 5.8g of monoisopropylamine, 0.13g of diisopropylamine, 1.6g isopropyl alcohol, 0.20g of n-propyl alcohol and 0.6g of a hydrocarbon oil were obtained, corresponding to a propylene conversion of 29% and a combined isopropylamines yield of 69%, propyl alcohols yield of 21% and oil yield of 10%.

Example 12

A mixture of 45.1g (0.5 mole) of dimethylamine, 14.0g (0.5 mole) of ethylene and 16.0g (0.3 mole) of ammonium chloride were charged to a 300cm$^3$ stainless steel autoclave. The autoclave was heated to 325° C and held there with agitation. The maximum system pressure was 235.4 bar (3,400 psig). A total of 2.18g of dimethylethylamine and 2.35g of diethylmethylamine were obtained, corresponding to an ethylene conversion of 30.1% and a combined dimethylethyl-amine and diethylmethylamine yield of 55.6%.

Example 13

A continuous reactor system was used consisting of a reactor 30.5 cm (12-inches) long by 1.59 cm (0.625-inches) I.D. A thermowell of 0.64 cm (0.25-inches) O.D. was mounted coaxially inside the reactor. The catalyst was held in the reactor by plugs of glass wool on either side of the catalyst, and the reactor tube was electrically heated.

The catalyst employed was prepared by dissolving 0.1 moles of ammonium iodide in 1.8 moles of methanol and injecting the solution into an evacuated flask containing 25 grams of carbon (Darco LI-100). The carbon was transferred in air into an evacuated desiccator where the methanol was vaporized at 70° C under 0.13 mbar (0.1 mmHg). This catalyst (36.7% $NH_4I$ on carbon, based on the weight of the combined catalyst system) was loaded into the reactor described above and dried under a nitrogen purge; first, at 70° C for one hour then at 390° C for an additional hour.

Ethylene and anhydrous liquid ammonia were fed from individual tanks by metering valves through flow meters and then mixed prior to vaporization in a coil of tubing immersed in hot silicon-oil maintained at 200° C. The molar feed ratio of ammonia and ethylene was 6.7 to 1.0. After seven hours of continuous running at 350° C and 25.1 bar (350 psig) of system pressure at a throughput of 14,000 GHSV, the total product was analyzed and found to contain monoethylamine in 98% yield and at a 7.7% conversion of ethylene. The product stream was trapped in a 3-liter cylinder chilled to -78° C and analyzed gas chromatographically.

For comparison purposes, carbon (Darco LI-100; 25g) was charged untreated (no ammonium iodide), in place of the above-described catalyst system, into the reactor system described above. The mixed ammonia and ethylene feed was vaporized and passed through the reactor tube maintained at 320° C and a pressure of 350 psig and at 14,000 GHSV. The resulting product contained only ammonia and ethylene and no amine, demonstrating that no amine is formed in the absence of ammonium halide.

Additionally, for comparison purposes, pure carbon (Westvaco; type-WVB) was charged (12.2g), in place of the catalyst system, into the reactor described above, and the $NH_3/C_2H_4$ gas mixture at a mole ratio of 11.8 to 1 was passed through the carbon bed in the reactor at a velocity of 1550 GHSV. The reactor was maintained at 14.8 bar (200 psig) and 190° C. No amine product was found.

## Example 14

Unsupported, solid, 100% ammonium iodide (25g) (12-18 mesh) was loaded as a catalyst into the reactor system described in Example 13.

A gaseous mixture of ammonia and ethylene (molar ratio 11.8:1.0) was passed over and through the salt-catalyst at 176° C and 12.7 bar (170 psig) of system pressure at a space velocity of 1,550 GHSV. The product gases were condensed in an evacuated cylinder chilled to -78° C. The product was analyzed by gas chromatography. The resulting product contained monoethylamine formed at an ethylene conversion of 40% in 99+% yield.

## Example 15

20 grams of a catalyst consisting of 28.5% (based on weight of catalyst system) $NH_4I$ on carbon (Westvaco; type-WVB) was placed into a reactor as described in Example 13 where it was heated and dried at 70° C with a $N_2$ stream passing over the catalyst for one hour. This was followed by increasing the temperature to 290° C and maintaining it there for one hour.

A liquid mixture of ammonia containing 1% propylene (mole ratio 256:1/$NH_3$:$C_3H_6$) was vaporized at 250° C and the hot gases were passed through the catalyst bed at 2000 GHSV. The reactor was maintained at 300° C and 7.2 bar (1090 psig). The product mixture contained propylamines in the molar ratio of 2.5:1.0 (n-propyl to isopropyl). The amines were produced in 2% conversion of propylene and a 80+% yield.

## Example 16

A catalyst as described in Example 13 was placed in a reactor as described in Example 13 where it was heated and dried at 70° C with a nitrogen purge for one hour and at 290° C for an additional hour.

A liquid mixture of ammonia containing 5 weight percent of dissolved ethylene (mole ratio 31.3:l.0/$NH_3$:$C_2H_4$) was vaporized and the reactant gas fixture at 1550 GHSV was passed through the catalyst bed maintained at 300° C and 13.1 bar (175 psig) pressure. The product mixture contained only monethylamine and unconverted ammonia and ethylene. The amine was formed at a 2% conversion of ethylene and a 99+% yield.

The following Example is specific to the incorporation of a promoter (i.e., $CrCl_3$) in a silica-supported $NH_4I$ vapor phase catalyst.

## Example 17

The reactor used for this work consisted of a 3m (10 ft.) coil of 316-SS tube 0.81 mm (0.032") wall thickness. The catalyst (60-80 mesh) was packed into the tube coil by suction and both ends were plugged with glass wool. The coil was heated by immersing it in a eutectic salt bath maintained at reaction temperature.

The catalyst employed was prepared by vacuum impregnation of Armak Silica (Si-5P) with 29% ammonium iodide/1% chromium chloride based on the weight of the total catalyst system (active catalyst, promoter and support), followed by drying under nitrogen at 100° C.

The reaction product was sampled by scrubbing the product stream with water (10° C-0° C) and condensing the water insoluble fraction in a dry-ice/acetone trap. No non-condensables were generated. The products were analyzed by gas chromatography.

A 1.5/1 molar mixture of ammonia/isobutylene was fed at a rate of 2 grams/minute (GHSV = 2,255 $hr^{-1}$) to the above-described reactor containing 40 $cm^3$ of catalyst maintained at 275° C and a pressure of 263 bar (3,800 psi) over a period of 3.53 hours; 425 grams of reactants were passed over the catalyst. A total of 53.0g of t-butylamine was obtained, corresponding to an isobutylene conversion of 13.8% and a t-butylamine yield of 100%. No coking or deactivation of the catalyst was noted and no hydrocarbon oil was formed.

## Claims

**1.** A process for producing amines, characterized by reacting ammonia or a primary or secondary amine with an aliphatic monoolefin having from 2 to 8 carbon atoms at a temperature ranging from 170° to 450° C, at a pressure of from about atmospheric to 587.1 bar (8500 psig) and in the presence of an ammonium halide-containing catalyst.

**2.** The process of Claim 1 wherein a promoter comprising a transition metal halide or ammonium salt of an inorganic oxyacid is present in an amount to improve the activity of the catalyst.

**3.** The process of Claim 1 wherein the reaction is continuous.

**4.** The process of Claim 1 wherein the temperature ranges from 250 to 350° C and the pressure ranges from 83.8 bar (1200 psig) to 276.9 bar (4000 psig).

**5.** The process of Claim 1 wherein said ammonium halide is ammonium iodide, ammonium bromide or ammonium chloride.

**6.** The process of Claim 1 wherein said ammonium halide is mounted on an inert, porous support.

**7.** The process of Claim 1 wherein said ammonium halide is dissolved or suspended in a solvent.

**8.** The process of Claim 7 where the solvent is water.

**9.** The process of Claim 5 wherein the temperature ranges from 250 to 350° C and the pressure ranges from 83.8 bar (1200 psig) to 276.9 bar (4000 psig).

**10.** The process of Claim 9 wherein the reactants are ammonia or a primary or secondary alkylamine having from 1 to 4 carbon atoms and a monoolefin having from 2 to 4 carbon atoms.

**11.** The process of Claim 10 wherein said ammonium halide is dissolved or suspended in a solvent.

**12.** The process of Claim 11 wherein said solvent is water.

**13.** The process of Claim 10 wherein said ammonium halide is mounted on an inert, porous support.

**14.** The process of Claim 13 wherein said support is silica.

**15.** The process of Claim 13 wherein the reaction is continuous.

**16.** The process of Claim 14 wherein the reaction is continuous.

**17.** The process of Claim 9 wherein a promoter comprising a transition-metal halide or ammonium salt of an inorganic oxyacid is present in an amount to improve the activity of the catalyst.

**18.** The process of Claim 17 wherein the promoter is present in an amount of 1 to 0.01 parts by weight per part by weight of active catalyst.

**19.** The process of Claim 18 wherein the promoter is mounted on an inert, porous support.

**20.** The process of Claim 19 wherein the catalyst is ammonium iodide and the promoter is chromium chloride.

**Revendications**

**1.** Procédé de production d'amines, caractérisé en ce qu'il consiste à faire réagir de l'ammoniac ou une amine primaire ou secondaire avec une mono-oléfine aliphatique ayant 2 à 8 atomes de carbone à une température comprise dans l'intervalle de 170° à 450° C, sous une pression allant d'approximativement la pression atmosphérique à 587,1 bars (8500 lb/in$^2$ au manomètre) et en présence d'un catalyseur contenant un halogénure d'ammonium.

**2.** Procédé suivant la revendication 1, dans lequel un promoteur comprenant un halogénure d'un métal de transition ou un sel d'ammonium d'un oxacide inorganique est présent en une quantité provoquant une amélioration de l'activité du catalyseur.

**3.** Procédé suivant la revendication 1, dans lequel la réaction est continue.

**4.** Procédé suivant la revendication 1, dans lequel la température est comprise dans l'intervalle de 250 à 350° C et la pression est comprise dans l'intervalle de 83,8 bars (1200 lb/in$^2$ au manomètre) à 276,9 bars (4000 lb/in$^2$ au manomètre).

**5.** Procédé suivant la revendication 1, dans lequel l'halogénure d'ammonium est l'iodure d'ammonium, le bromure d'ammonium ou le chlorure d'ammonium.

**6.** Procédé suivant la revendication 1, dans lequel l'halogénure d'ammonium est monté sur un support poreux inerte.

**7.** Procédé suivant la revendication 1, dans lequel l'halogénure d'ammonium est dissous ou mis en suspension dans un solvant.

**8.** Procédé suivant la revendication 7, dans lequel le solvant est l'eau.

**9.** Procédé suivant la revendication 5, dans lequel la température est comprise dans l'intervalle de 250 à 350 °C et la pression est comprise dans l'intervalle de 83,8 bars (1200 lb/in$^2$ au manomètre) à 276,9 bars (4000 lb/in$^2$ au manomètre).

**10.** Procédé suivant la revendication 9, dans lequel les corps réactionnels sont l'ammoniac ou une alkylamine primaire ou secondaire ayant 1 à 4 atomes de carbone et une mono-oléfine ayant 2 à 4 atomes de carbone.

**11.** Procédé suivant la revendication 10, dans lequel l'halogénure d'ammonium est dissous ou mis en suspension dans un solvant.

**12.** Procédé suivant la revendication 11, dans lequel le solvant est l'eau.

**13.** Procédé suivant la revendication 10, dans lequel l'halogénure d'ammonium est monté sur un support poreux inerte.

**14.** Procédé suivant la revendication 13, dans lequel le support est la silice.

**15.** Procédé suivant la revendication 13, dans lequel la réaction est continue.

**16.** Procédé suivant la revendication 14, dans lequel la réaction est continue.

**17.** Procédé suivant la revendication 9, dans lequel un promoteur comprenant un halogénure d'un métal de transition ou un sel d'ammonium d'un oxacide inorganique est présent en une quantité améliorant l'activité du catalyseur.

**18.** Procédé suivant la revendication 17, dans lequel le promoteur est présent en une quantité de 1 à 0,01 partie en poids par partie en poids de catalyseur actif.

**19.** Procédé suivant la revendication 18, dans lequel le promoteur est monté sur un support poreux inerte.

**20.** Procédé suivant la revendication 19, dans lequel le catalyseur est l'iodure d'ammonium et le promoteur est le chlorure de chrome.

## Ansprüche

**1.** Verfahren zur Herstellung von Aminen, dadurch **gekennzeichnet,** daß man Ammoniak oder ein primäres oder sekundäres Amin mit einem aliphatischen Monoolefin mit 2 biS 8 Kohlenstoffatomen bei einer Temperatur im Bereich von 170 biS 450 °C, bei einem Druck von etwa Atmosphärendruck bis 587,1 bar (8500 psig) und in Gegenwart eines Ammoniumhalogenid enthaltenden Katalysators umsetzt.

**2.** Verfahren nach Anspruch 1, dadurch **gekennzeichnet,** daß ein Promotor, umfassend ein Übergangsmetall-Halogenid oder ein Ammoniumsalz einer anorganischen Oxysäure, in einer Menge vorhanden ist, daß die Aktivität des Katalysators verbessert wird.

**3.** Verfahren nach Anspruch 1, dadurch **gekennzeichnet,** daß die Reaktion kontinuierlich ist.

**4.** Verfahren nach Anspruch 1, dadurch **gekennzeichnet,** daß die Temperatur im Bereich von 250 bis 350 °C liegt und daß der Druck im Bereich von 83,8 bar (1200 psig) bis 276,9 bar (4000 psig) liegt.

**5.** Verfahren nach Anspruch 1, dadurch **gekennzeichnet,** daß das Ammoniumhalogenid Ammoniumiodid, Ammoniumbromid oder Ammoniumchlorid ist.

**6.** Verfahren nach Anspruch 1, dadurch **gekennzeichnet,** daß das Ammoniumhalogenid auf einem inerten, porösen Träger aufgebracht ist.

**7.** Verfahren nach Anspruch 1, dadurch **gekennzeichnet,** daß das Ammoniumhalogenid in einem Lösungsmittel aufgelöst oder suspendiert ist.

**8.** Verfahren nach Anspruch 7, dadurch **gekennzeichnet**, daß das Lösungsmittel Wasser ist.

**9.** Verfahren nach Anspruch 5, dadurch **geken-**

**nzeichnet,** daß die Temperatur im Bereich von 250 bis 350 °C liegt und daß der Druck im Bereich von 83,8 bar (1200 psig) bis 276,9 bar (4000 psig) liegt.

**10.** Verfahren nach Anspruch 9, dadurch **gekennzeichnet,** daß die Reaktanten Ammoniak oder ein primäres oder sekundäres Alkylamin mit 1 bis 4 Kohlenstoffatomen und ein Monoolefin mit 2 bis 4 Kohlenstoffatomen sind.

**11.** Verfahren nach Anspruch 10, dadurch **gekennzeichnet,** daß das Ammoniumhalogenid in einem Lösungsmittel aufgelöst oder suspendiert ist.

**12.** Verfahren nach Anspruch 11, dadurch **gekennzeichnet,** daß das Lösungsmittel Wasser ist.

**13.** Verfahren nach Anspruch 10, dadurch **gekennzeichnet,** daß das Ammoniumhalogenid auf einem inerten porösen Träger aufgebracht ist.

**14.** Verfahren nach Anspruch 13, dadurch **gekennzeichnet,** daß der Träger Siliciumdioxid ist.

**15.** Verfahren nach Anspruch 13, dadurch **gekennzeichnet,** daß die Reaktion kontinuierlich ist.

**16.** Verfahren nach Anspruch 14, dadurch **gekennzeichnet,** daß die Reaktion kontinuierlich ist.

**17.** Verfahren nach Anspruch 9, dadurch **gekennzeichnet,** daß ein Promotor, umfassend ein Übergangsmetall-Halogenid oder ein Ammoniumsalz einer anorganischen Oxysäure, in einer Menge vorhanden ist, daß die Aktivität des Katalysators verbessert wird.

**18.** Verfahren nach Anspruch 17, dadurch **gekennzeichnet,** daß der Promotor in einer Menge von 1 bis 0,01 Gew.-Teilen pro Gew.-Teil aktiver Katalysator vorhanden ist.

**19.** Verfahren nach Anspruch 18, dadurch **gekennzeichnet,** daß der Promotor auf einem inerten, porösen Träger aufgebracht ist.

**20.** Verfahren nach Anspruch 19, dadurch **gekennzeichnet,** daß der Katalysator Ammoniumiodid ist und daß der Promotor Chromchlorid ist.